# EUROPEAN PATENT APPLICATION

(11) **EP 4 722 367 A1**
(43) Date of publication of application: **08.04.2026**
(21) Application number: 24815745.5
(22) Date of filing: 14.05.2024
(51) Int. Cl.: C12N 15/113, C12N 15/63, A61K 31/7088

(54) **MRNA STRUCTURE PLATFORM FOR PROTEIN EXPRESSION WITH HIGH EFFICIENCY**

(30) Priority: 26.05.2023 KR 20230068278
(71) Applicant: Korea Research Institute of Bioscience and Biotechnology, Daejeon 34141 (KR)
(72) Inventor: CHO, Sungchan, Daejeon 34141 (KR); JO, Min Ju, Daejeon 34141 (KR); KIM, Seyoung, Daejeon 34141 (KR); JEONG, Min Sun, Daejeon 34141 (KR); HAM, Youngwook, Daejeon 34141 (KR)
(74) Representative: KATZAROV S.A.
(86) International application number: PCT/KR2024/006580
(87) International publication number: WO 2024/248367

(57) **Abstract**

The present invention relates to an mRNA structure platform capable of expressing a protein with high efficiency.

## Description

### [Technical Field]

### CROSS-REFERENCE TO RELATED APPLICATION

This application claims the benefit of priority to Korean Patent Application No. 10-2023-0068278, filed in the Korean Intellectual Property Office on May 26, 2023, the entire contents of which are incorporated herein by reference.

### Technical Field

The present disclosure relates to an mRNA construct platform capable of expressing a protein with high efficiency.

### [Background Art]

As COVID-19, an infectious disease caused by the novel coronavirus SARS-CoV-2 that emerged in 2019, rapidly spread worldwide, vaccine development was competitively pursued at the national level to prevent further spread and reduce mortality. Among various vaccine development strategies, the mRNA vaccine that is classified as a relatively new strategy was approved for use in the shortest period of time and has been administered to date. The mRNA vaccine is a nucleic acid-based vaccine that enhances adaptive immunity by using artificially synthesized mRNA (messenger RNA, a blueprint containing antigen protein information), and mRNA containing viral antigen genes is synthesized ex vivo and purified to high purity, then administered to the human body in a form encapsulated in lipid nanoparticles (LNPs), and the antigen expressed by immune cells is presented outside the cells to induce diverse antigen-specific adaptive immune responses.

In December 2020, the mRNA vaccines of Moderna (mRNA-1273) and Pfizer-BioNTech (BNT162b2) received emergency use authorization in major countries including the United States, and their remarkable efficacy of over 95% was identified in large-scale clinical trials involving more than 30,000 participants, and this is in stark contrast to traditional vaccines that remain at the level of 50 to 70%. Despite the unprecedented success of mRNA vaccines, their efficacy does not last long, making additional booster doses essential. Furthermore, as time passes after vaccination, neutralizing antibody titers and vaccine efficacy decrease, and it has been reported that for both the Modema and Pfizer-BioNTech mRNA vaccines, neutralizing antibody titers decrease more than sixfold 6 to 8 months after the second dose.

In addition, the continuous emergence of mutant viruses due to the prolonged COVID-19 pandemic has become another cause of reduced efficacy of mRNA vaccines. Because SARS-CoV-2 is an RNA virus, numerous mutations naturally occur during the replication process, and when mutations occur in areas, in which neutralizing antibodies act, the neutralizing antibodies are unable to function, resulting in a reduction or loss of vaccine efficacy.

Accordingly, to enhance the durability of mRNA vaccine efficacy, it is necessary to develop advanced mRNA constructs that enable antigen expression in antigen-presenting immune cells to be higher and more sustained than that of first-generation mRNA vaccines. In particular, it is considered important for the activation of adaptive immunity that not only inactive antigen-presenting immune cells, but also activated antigen-presenting immune cells exhibit sustained high-level expression even after migrating to lymph nodes.

Meanwhile, active research is currently being conducted on the application of the mRNA not only for vaccines against infectious diseases but also for the development of cancer vaccines, and it is expected that, in the future, its scope of use will further expand to the development of therapeutics for various rare genetic diseases and intractable diseases.

The approach of delivering disease-causing genes in the form of mRNA into cells and organisms to supplement the amount or activity of proteins and thus produce therapeutic effects is most suitable for genetic diseases caused by a decrease in the amount or activity of genes. of which correlation with the disease has been clearly verified, particularly, for monogenic diseases, in which defects in a single gene are the cause.

The method of developing vaccines or therapeutics using mRNA as described above directly supplies disease-causing genes, and thus is a highly target-specific approach, and because it utilizes RNA, it does not integrate into the genome and enables only transient expression, so that high safety is ensured, and according to this method, when the mRNA construct and delivery module are established, various disease-related genes may be easily applied. Moreover, because production and processing are efficient and the overall development period is short, economical drug development may be achieved, which is advantageous for the development of low-cost drugs, and this approach may be highly suitable for the development of personalized therapeutics in the future.

Recently, several gene therapies using adeno-associated virus (AAV) in an in vivo delivery method have been approved as new drugs. Representative examples include Zolgensma, a therapeutic for spinal muscular atrophy, Zynteglo, a therapeutic for anemia, and Hemgenix, a therapeutic for hemophilia, and their prices reach an astronomical level of 2.5 to 4.5 billion KRW per dose. Despite the advantage of providing therapeutic effects for several years with a single administration, limitations have been recognized in that the packaging capacity of the AAV vector imposes restrictions on gene size, the drug price remains unrealistically high, immune responses are not negligible, and in patients previously exposed to the AAV virus, neutralizing antibodies are formed, resulting in a loss of efficacy.

In contrast, mRNA-based therapeutics have no limitation on the size of the applied gene in theory, offer high safety, allow relatively low drug prices through economical pharmaceutical production, and have no restriction on patient groups, thereby complementing the weaknesses of AAV-based therapeutics. Despite these advantages of mRNA-based therapeutics, for mRNA to be successful as a therapeutic, it is essential that disease-related genes be expressed at high levels and in a sustained manner over a long period of time, because unlike vaccines, which may induce immune memory in vivo by expressing sufficient antigens with a single administration through immune responses, in the case of therapeutics, continuously high expression of disease-related proteins is important for producing therapeutic effects.

Accordingly, the development of an mRNA construct platform capable of expressing proteins with high efficiency is not only an essential process that may be applied to the effective and rapid development of mRNA vaccines to overcome not only high-risk viruses, such as SARS-CoV-2, Zika, influenza, SFTS, and Dengue, but also newly emerging viral infectious diseases in the future, but also that enables the development of mRNA-based gene therapeutics.

### [Disclosure]

### [Technical Problem]

An aspect of the present disclosure provides a novel mRNA construct that may be applied as a platform capable of expressing a target protein with high efficiency.

An aspect of the present disclosure also provides a pharmaceutical composition including the novel mRNA construct.

### [Technical Solution]

To achieve the above object, an aspect of the present disclosure provides an mRNA construct comprising: a nucleotide sequence encoding a target protein; a nucleotide sequence of the ζ-globin 3'-UTR disposed downstream of the nucleotide sequence encoding the target protein; and a nucleotide sequence including (A₆₀G)n repeat units disposed downstream of the nucleotide sequence of the ζ-globin 3'-UTR, wherein n is an integer from 1 to 3.

The mRNA construct may further comprise a nucleotide sequence derived from a black beetle virus (BBV) or a peste des petits ruminants virus (PPRV), disposed upstream of the nucleotide sequence encoding the target protein.

The mRNA construct may further comprise a nucleotide sequence of an internal ribosome entry site (IRES) of an encephalomyocarditis virus (EMCV) between a downstream side of the nucleotide sequence encoding the target protein and an upstream side of the nucleotide sequence of the ζ-globin 3'-UTR.

In addition, to achieve the above object, another aspect of the present disclosure provides a pharmaceutical composition comprising the mRNA construct.

The pharmaceutical composition may be a vaccine or a gene therapeutic agent.

### [Advantageous Effects]

The mRNA construct newly designed and derived in the present disclosure exhibits far more excellent expression efficiency in cells compared with the mRNA constructs of Modema or Pfizer-BioNTech that have been commercially available, and thus may be usefully applied as a platform technology that may be widely utilized in the development of mRNA-based vaccines and therapeutics.

However, the effects of the present disclosure are not limited to those mentioned above, and other effects not mentioned will be clearly understood by those skilled in the art from the following description.

### [Description of Drawings]

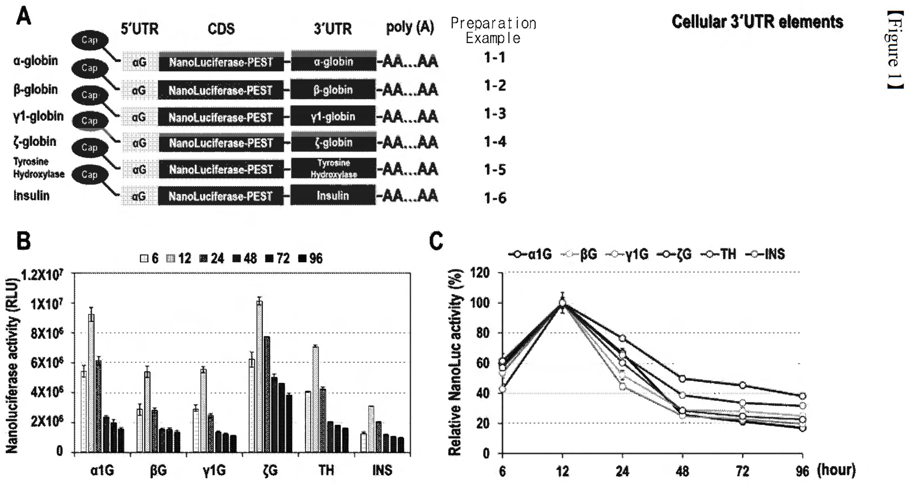

FIG. 1 illustrates (A) the design of mRNA constructs by applying nucleotide sequences of various cellular genes to the 3'-UTR to derive an optimal 3'-UTR, (B) a result obtained by measuring the activity of nanoluciferase over time after introducing the mRNA constructs into 293T cells by transfection, and (C) a result obtained by correcting the activity of the nanoluciferase with the 12-hour result and then analyzing changes in relative activity.

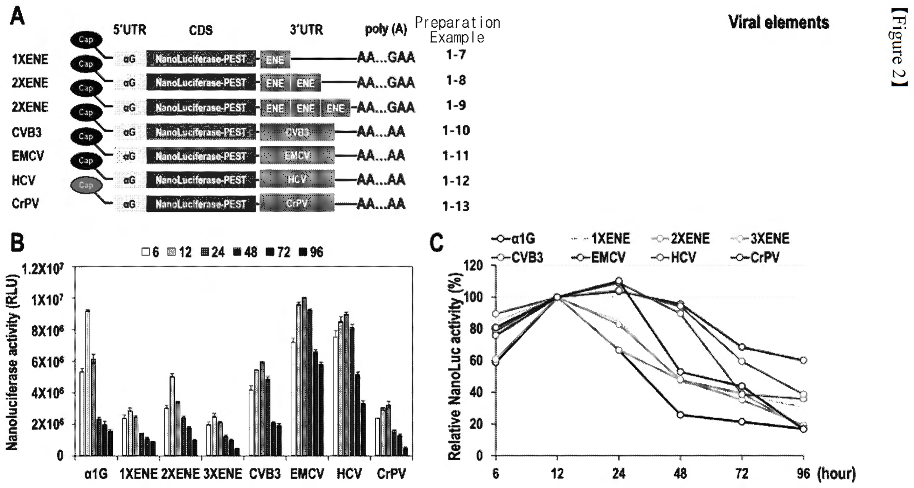

FIG. 2 illustrates (A) the design of mRNA constructs by applying nucleotide sequences derived from various viruses to the 3'-UTR to derive an optimal 3'-UTR, (B) a result obtained by measuring the activity of nanoluciferase over time after introducing the mRNA constructs into 293T cells by transfection, and (C) a result obtained by correcting the activity of the nanoluciferase with the 12-hour result and then analyzing changes in relative activity.

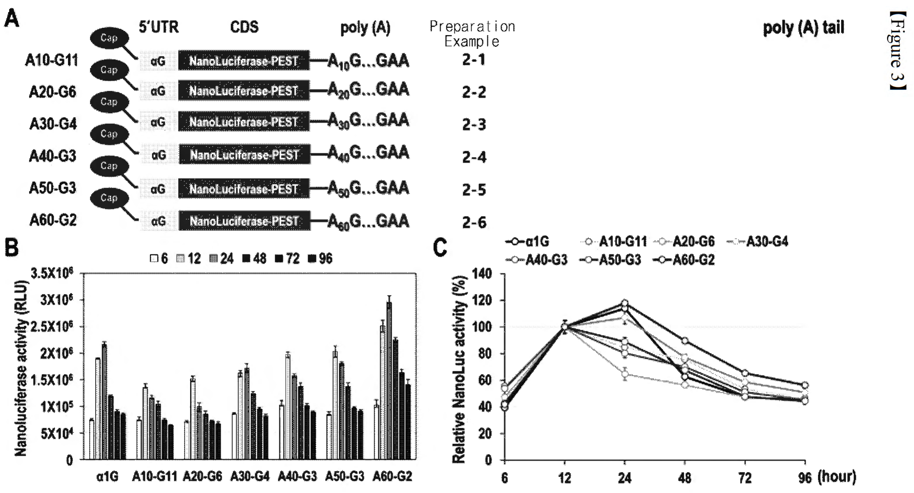

FIG. 3 illustrates (A) the design of mRNA constructs by applying nucleotide sequences in which guanines are inserted into the poly(A) tail in various numbers and intervals to derive an optimal poly(A) tail, (B) a result obtained by measuring the activity of nanoluciferase over time after introducing the mRNA constructs into 293T cells by transfection, and (C) a result obtained by correcting the activity of the nanoluciferase with the 12-hour result and then analyzing changes in relative activity.

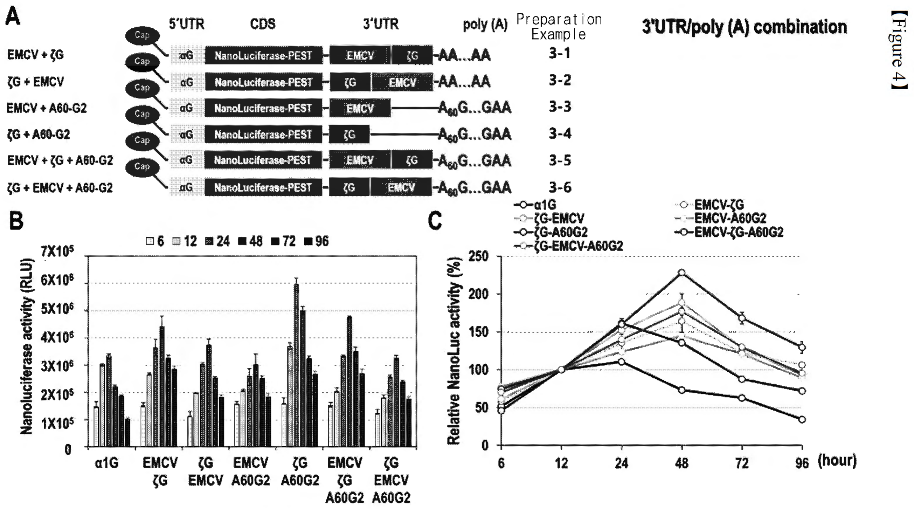

FIG. 4 illustrates (A) the design of mRNA constructs by applying, alone or in combination, a nucleotide sequence of the 3'-UTR of the ζ-globin or a nucleotide sequence of the IRES of the EMCV to the 3'-UTR, and by applying A60G2 as the poly(A) tail, (B) a result obtained by measuring the activity of nanoluciferase over time after introducing the mRNA constructs into 293T cells by transfection, and (C) a result obtained by correcting the activity of the nanoluciferase with the 12-hour result and then analyzing changes in relative activity.

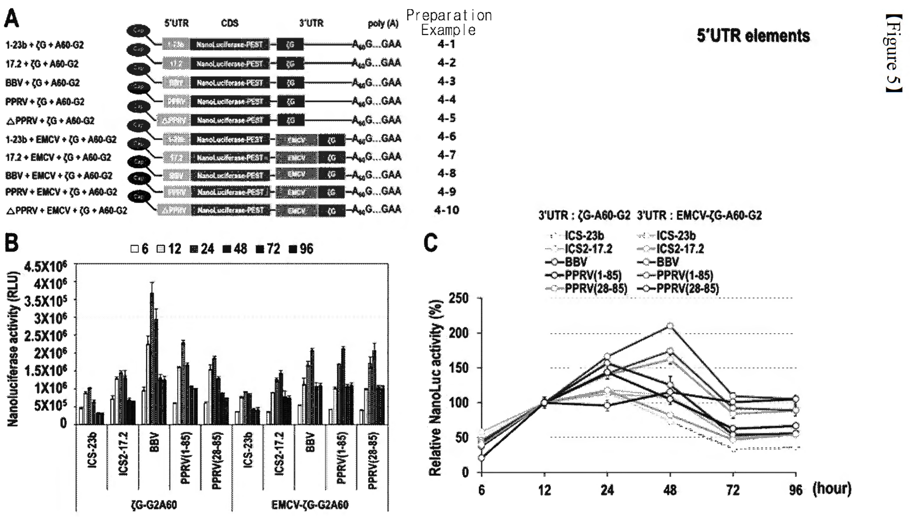

FIG. 5 illustrates (A) the design of mRNA constructs by applying, to the 3'-UTR, a nucleotide sequence of the 3'-UTR of the ζ-globin or a combination of a nucleotide sequence of the IRES of the EMCV and a nucleotide sequence of the 3'-UTR of the ζ-globin, and by applying A₆₀G₂ as the poly(A) tail, while applying various nucleotide sequences to the 5'-UTR to derive an optimal 5'-UTR, (B) a result obtained by measuring the activity of nanoluciferase over time after introducing the mRNA constructs into 293T cells by transfection, and (C) a result obtained by correcting the activity of the nanoluciferase with the 12-hour result and then analyzing changes in relative activity.

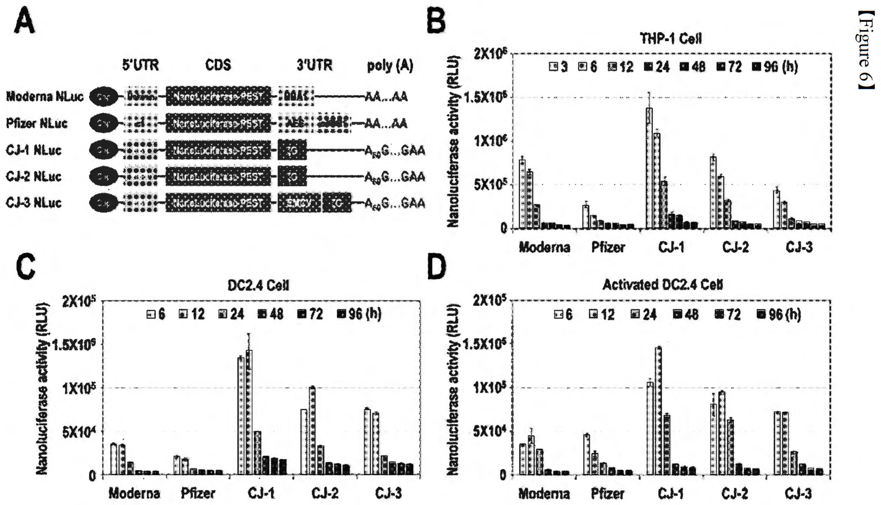

FIG. 6 illustrates (A) the selection of three mRNA constructs (CJ-1, BBV+ζG+ A₆₀G₂; CJ-2, PPRV+ζG+A₆₀G₂; and CJ-3, BBV+EMCV+ζG+ A₆₀G₂ that showed the highest expression efficiency in FIG. 5, and the additional preparation of mRNA constructs used for coronavirus mRNA vaccine development by Modema and Pfizer-BioNTech, followed by (B) a result obtained by introducing the mRNA constructs into THP-1 cells, which are monocyte-line immune cells, (C) a result obtained by introducing the mRNA constructs into mouse DC2.4 cells, which are dendritic cell-line cells, and (D) a result obtained by introducing the mRNA constructs into activated DC2.4 cells that were activated with LPS, and then measuring the activity of nanoluciferase over time.

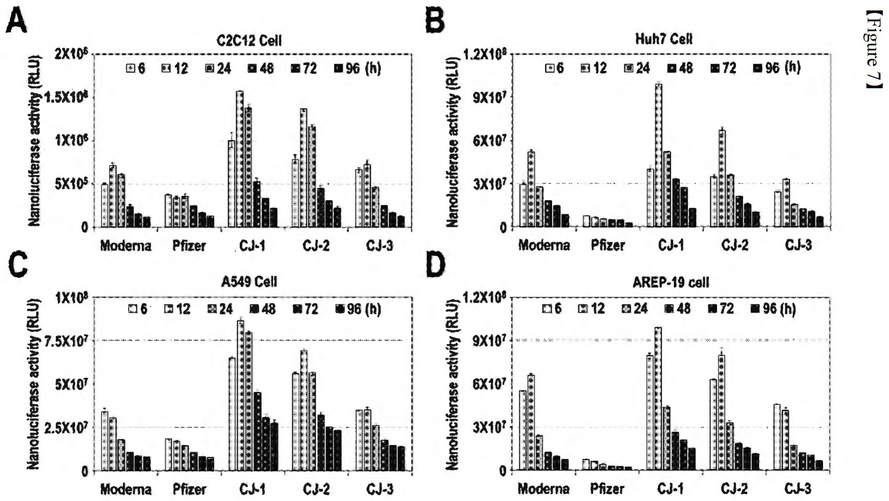

FIG. 7 illustrates results obtained by introducing the three mRNA constructs (CJ-1, CJ-2, and CJ-3) into (A) C2C12 cells, which are representative mouse muscle cells, (B) Huh-7 cells, which are representative hepatocytes, (C) A549 cells, which are representative lung cells, (D) ARPE-19 cells, which are representative retinal cells, SH-SY5Y cells (ATCC) and Neuro-2a cells (ATCC), which are representative neuronal cells, and 293T cells, and then measuring the activity of nanoluciferase over time.

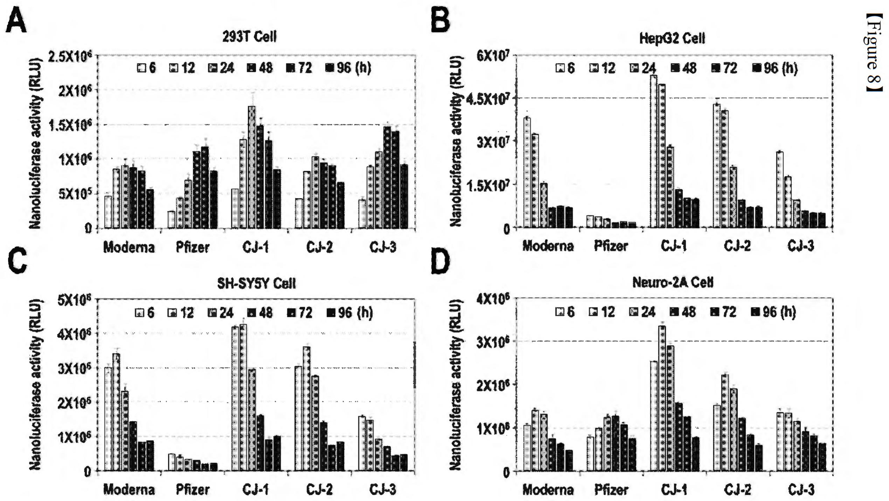

FIG. 8 illustrates results obtained by introducing the three mRNA constructs (CJ-1, CJ-2, and CJ-3) into (A) 293T cells, (B) HepG2 cells, which are another type of representative hepatocytes, (C) SH-SY5Y cells, which are representative neuronal cells, and (D) Neuro-2a cells, which are representative neuronal cells, and then measuring the activity of nanoluciferase over time.

### [Mode for Invention]

Hereinafter, the present disclosure will be described in detail.

### 1. Novel mRNA construct

An aspect of the present disclosure provides a novel mRNA construct.

The mRNA construct of the present disclosure includes a nucleotide sequence that encodes a target protein; a nucleotide sequence of the 3'-untranslated region (UTR) of the ζ-globin; and a nucleotide sequence including (A₆₀G)n repeat units.

The target protein refers to a protein that is intended to be expressed through the mRNA construct of the present disclosure. Specifically, the target protein may refer to a protein that is directly translated and expressed from the mRNA construct by the intrinsic translation system of the cell after the mRNA construct of the present disclosure is introduced into the cell. Accordingly, the "nucleotide sequence that encodes a target protein" included in the mRNA construct of the present disclosure may refer to a coding sequence (CDS), in which introns are removed and only exons are present, so that it may be directly translated into the target protein.

The ζ-globin is a human HBZ gene, and is one of the five genes belonging to the human α-globin gene cluster. The nucleotide sequence of the 3'-UTR of the ζ-globin refers to the nucleotide sequence of the 3'-UTR that is included in the mRNA generated in a process of expressing the human HBZ gene. In particular, the nucleotide sequence of the 3'-UTR of the ζ-globin may include the nucleotide sequence of SEQ ID NO: 6 or a variant thereof, and specifically, the nucleotide sequence of the 3'-UTR of the ζ-globin may be constituted by the nucleotide sequence of SEQ ID NO: 6 or a variant thereof. The variant of the nucleotide sequence of SEQ ID NO: 6 refers to those having a sequence homology of 80% or more, 90% or more, 95% or more, 96% or more, 97% or more, 98% or more, or 99% or more with the nucleotide sequence of SEQ ID NO: 6, but is not limited thereto, and refers to all nucleotide sequences that function to improve the stability and expression efficiency of mRNA, like the nucleotide sequence of SEQ ID NO: 6.

The nucleotide sequence of the 3'-UTR of the ζ-globin may be disposed downstream of the nucleotide sequence that encodes the target protein, and in particular, may be operably linked to the nucleotide sequence that encodes the target protein.

The nucleotide sequence including the (A₆₀G)n repeat units is a variant of a poly(A) tail, and the nucleotide sequence including the (A₆₀G)ₙ repeat units may have a length of 115 nt to 125 nt, for example, may have a length of 117 nt to 123 nt, and in particular, the nucleotide sequence including the (A₆₀G)ₙ repeat units may have a length of 121 nt. Accordingly, n may be an integer from 1 to 3, for example, may be an integer from 1 to 2, and in particular, n may be 1. Specifically, the nucleotide sequence including the (A₆₀G)ₙ repeat units may be the nucleotide sequence of SEQ ID NO: 21 (A₅₇GA₆₀GAA).

The nucleotide sequence including the (A₆₀G)ₙ repeat units may be disposed downstream of the nucleotide sequence of the 3'-UTR of the ζ-globin, and in particular, may be operably linked to the nucleotide sequence that encodes the target protein and the nucleotide sequence of the 3'-UTR of the ζ-globin.

Meanwhile, the mRNA construct of the present disclosure may further include a nucleotide sequence of the 5'-UTR of a black beetle virus (BBV) or a nucleotide sequence of the 5'-UTR of a peste des petits ruminants virus (PPRV). The nucleotide sequence of the 5'-UTR of the BBV is a sequence that is derived from a virus that infects black beetles, and is located upstream of a coding sequence, so that the translation efficiency of a protein is improved. In particular, the nucleotide sequence of the 5'-UTR of the BBV is much shorter than an IRES nucleotide sequence, which is known as a cap-independent translation mechanism, and exhibits higher protein translation efficiency. In addition, the PPRV belongs to the genus Morbillivirus under the family Paramyxoviridae, and is a peste des petits ruminants virus that mainly infects goats and sheep. The nucleotide sequence of the 5'-UTR of the PPRV is presumed to improve protein translation efficiency as it is located upstream of the F gene of the PPRV and complementarily binds to 18S rRNA.

In particular, the nucleotide sequence of the 5'-UTR of the BBV may include the nucleotide sequence of SEQ ID NO: 24 or a variant thereof, and specifically, the nucleotide sequence of the 5'-UTR of the BBV may be constituted by the nucleotide sequence of SEQ ID NO: 24 or a variant thereof. In addition, the nucleotide sequence of the 5'-UTR of the PPRV may include the nucleotide sequence of SEQ ID NO: 25, the nucleotide sequence of SEQ ID NO: 26, or a variant thereof, and specifically, the nucleotide sequence of the 5'-UTR of the PPRV may be constituted by the nucleotide sequence of SEQ ID NO: 25, the nucleotide sequence of SEQ ID NO: 26, or a variant thereof. The variant of the nucleotide sequence of SEQ ID NOs: 24, 25, or 26 refers to those having a sequence homology of 80% or more, 90% or more, 95% or more, 96% or more, 97% or more, 98% or more, or 99% or more with the nucleotide sequence of SEQ ID NOs: 24, 25, or 26, but is not limited thereto, and refers to all nucleotide sequences that function to improve the stability and expression efficiency of mRNA, like the nucleotide sequence of SEQ ID NOs: 24, 25, or 26.

The nucleotide sequence of the 5'-UTR of the BBV or the nucleotide sequence of the 5'-UTR of the PPRV may be disposed upstream of the nucleotide sequence that encodes the target protein, and in particular, may be operably linked to the nucleotide sequence that encodes the target protein.

Furthermore, the mRNA construct of the present disclosure may further include a nucleotide sequence of the internal ribosome entry site (IRES) of the encephalomyocarditis virus (EMCV). The nucleotide sequence of the IRES of the EMCV may be constituted by the nucleotide sequence of SEQ ID NO: 13 or a variant thereof. The variant of the nucleotide sequence of SEQ ID NO: 13 refers to those having a sequence homology of 80% or more, 90% or more, 95% or more, 96% or more, 97% or more, 98% or more, or 99% or more with the nucleotide sequence of SEQ ID NO: 13, but is not limited thereto, and refers to all nucleotide sequences that function to improve the stability and expression efficiency of mRNA, like the nucleotide sequence of SEQ ID NO: 13.

The nucleotide sequence of the IRES of the EMCV may be disposed between a downstream side of the nucleotide sequence that encodes the target protein and an upstream side of the nucleotide sequence of the 3'-UTR of the ζ-globin, and in particular, may be operably linked to the nucleotide sequence that encodes the target protein and the nucleotide sequence of the 3'-UTR of the ζ-globin.

The mRNA construct of the present disclosure configured as described above has very excellent stability and expression efficiency in cells, and accordingly, may maintain a high expression state in the cells for a long period of time even after being introduced by transfection. In particular, it was identified that the mRNA construct of the present disclosure exhibits higher expression efficiency as compared to the conventional mRNA constructs of Modema or Pfizer-BioNTech, and it was also identified that the mRNA construct exhibits consistently high expression efficiency regardless of various types of cells, such as muscle cells, hepatocytes, lung cells, ocular cells, and neuronal cells (see FIGS. 6 to 8).

### Utilization of novel mRNA construct

As described above, the novel mRNA construct of the present disclosure exhibits high expression efficiency in cells, and thus may be for being introduced into cells by transfection for the expression of a target protein.

Furthermore, the novel mRNA construct of the present disclosure that may be introduced into cells by transfection as described above may be used as an active ingredient of a pharmaceutical composition depending on the target protein.

For example, when the target protein is a protein that is derived from a virus or bacterium, the pharmaceutical composition including the mRNA construct of the present disclosure may be a vaccine. In this case, a protein derived from a virus or bacterium, which is a target protein, is expressed at high efficiency by the novel mRNA construct in an individual, into which the pharmaceutical composition of the present disclosure is administered, and the protein derived from a virus or bacterium expressed in this way may be recognized as an antigen in the individual to induce an immune response, thereby functioning as a vaccine.

Furthermore, when the target protein is a causal protein that is deficient in a specific disease, such as cancer or a genetic disease, the pharmaceutical composition including the mRNA construct of the present disclosure may be a gene therapeutic agent. In this case, a causal protein that is deficient in the specific disease, which is a target protein, is expressed at high efficiency by the novel mRNA construct in an individual, into which the pharmaceutical composition of the present disclosure is administered, and the deficiency of the causal protein may be complemented for by the high-efficiency expression of the causal protein, so that the specific disease may be treated. Accordingly, any disease that may be treated by complementing a deficient gene may be treated by the pharmaceutical composition of the present disclosure without particular limitation.

In particular, diseases that are most suitable to be applied as gene therapeutic agents as described above are monogenic genetic diseases that occur due to a single gene defect, and for example, spinal muscular atrophy (SMA), which is a rare neuromuscular disease, is representative, and in addition, cystic fibrosis (CF), Duchenne muscular dystrophy (DMD), Friedreich's ataxia (FA), hemophilia, Rett syndrome, Fabry disease, and sickle cell disease may also be applicable.

Moreover, the mRNA construct of the present disclosure exhibits consistently high expression efficiency regardless of various types of cells, such as muscle cells, hepatocytes, lung cells, ocular cells, and neuronal cells, and thus may be applied to various cells, in which various diseases may be caused. Accordingly, when the mRNA construct of the present disclosure is used as an active ingredient of a gene therapeutic agent as described above, it may ensure a much more excellent therapeutic effect as compared to cases, in which mRNA constructs of Modema or Pfizer-BioNTech are used.

Meanwhile, when the mRNA construct of the present disclosure is used as an active ingredient of a pharmaceutical composition as described above, the pharmaceutical composition may further include a delivery means for delivering the mRNA construct of the present disclosure.

Representative examples of the delivery means may include nanoparticles, such as liposomes or lipid nanoparticles (LNPs), and the liposome or the LNP may include cationic lipids, non-cationic lipids, or neutral lipids, and may additionally include other lipids, such as polyethylene glycol (PEG) or cholesterol. Furthermore, exosomes may also be used as nanoparticles that serve as the delivery means as described above, and cationic biocompatible polymers or cationic peptides (e.g., protamine and cell penetrating peptides (CPPs)) that may electrostatically interact with the mRNA construct of the present disclosure to provide enhanced stability may also be used as another delivery means, but the present disclosure is not limited thereto, and any means known in the art, to which the present disclosure pertains, for delivering an mRNA construct may be used.

Furthermore, the pharmaceutical composition may additionally include one or more pharmaceutically acceptable carriers. The pharmaceutically acceptable carrier must be compatible with the active ingredient of the present disclosure, and may be selected from saline, sterile water, Ringer's solution, buffered saline, dextrose solution, maltodextrin solution, glycerol, ethanol, and a mixture of one or more of these components, and other common additives, such as antioxidants, buffers, and bacteriostatic agents, may be further added as needed. Furthermore, diluents, dispersants, surfactants, binders, and lubricants may be additionally added to formulate the composition into injectable preparations, such as aqueous solutions, suspensions, and emulsions. In particular, it is preferable to formulate and provide the preparation in a lyophilized form. For manufacturing a lyophilized preparation, methods that are conventionally known in the technical field, to which the present disclosure pertains, may be used, and stabilizers for lyophilization may also be additionally included. Furthermore, the preparation may be preferably formulated depending on each disease or component by using appropriate methods in the art, or by using the methods disclosed in Remington's Pharmaceutical Science (Mack Publishing Company, Easton, PA).

The content of the active ingredient included in the pharmaceutical composition, and the method of administration, may be determined by those skilled in the art based on the symptoms of an ordinary patient and the severity of the disease. Furthermore, the preparation may be formulated in various forms, such as powders, tablets, capsules, liquids, injections, ointments, and syrups, and may be provided in unit-dose or multi-dose containers, for example, sealed ampoules and vials.

Furthermore, the pharmaceutical composition may be administered orally or parenterally. The administration routes of the composition according to the present disclosure are not limited thereto, but, for example, may include intrabronchial, oral, intravenous, intramuscular, intraarterial, intramedullary, intradural, intracardiac, transdermal, subcutaneous, intraperitoneal, enteral, sublingual, or topical administration. The dosage of the composition according to the present disclosure may vary depending, for example, the patient's body weight, age, sex, health condition, diet, time and method of administration, excretion rate, or severity of the disease, and may be readily determined by those skilled in the art. Furthermore, for clinical administration, the pharmaceutical composition may be formulated into a suitable preparation by using known techniques.

Hereinafter, the present disclosure will be described in detail with reference to embodiments.

However, the following embodiments are provided to specifically illustrate the present disclosure, and the scope of the present disclosure is not limited by the following embodiments.

### [Example 1]

### Structure of novel mRNA construct

To derive a novel mRNA construct that has high stability and high translation efficiency in cells in a linear form, first, nanoluciferase, which has the highest luminescence and the smallest protein size, among reporter proteins, and a nanoluciferase-PEST fusion protein, in which PEST amino acids are fused to the C-terminus of the nanoluciferase, were selected as target proteins to be expressed. Furthermore, with respect to the nucleotide sequence of SEQ ID NO: 1 that encodes the nanoluciferase-PEST fusion protein, the nucleotide sequence of a 5'-UTR was disposed upstream thereof, and CleanCap (TriLink Inc.) in the form of Cap1 was disposed at a 5' distal end of the nucleotide sequence of the 5'-UTR, and a structure of the novel mRNA construct was specified in a form, in which the nucleotide sequence of a 3'-UTR and the nucleotide sequence of a poly(A) tail are sequentially disposed downstream thereof.

### [Example 2]

### Selection of optimal components of novel mRNA construct

### [2-1] Selection of optimal 3'-UTR

First, to derive an optimal 3'-UTR, in the mRNA construct specified in Embodiment 1, the nucleotide sequence of the 5'-UTR was fixed as the nucleotide sequence of the 5'-UTR of human α-globin (the nucleotide sequence of SEQ ID NO: 2), and the nucleotide sequence of the poly(A) tail was fixed as a 117-nt poly(A) sequence, and by applying various nucleotide sequences of 3'-UTRs disclosed in Table 1 as the nucleotide sequence of the 3'-UTR, six types of mRNA constructs of Preparation Examples 1-1 to 1-6 as illustrated in FIG. 1A were designed.

**[Table 1]**

| **Applied preparation example #** | **Name** | **nucleotide sequence (5'->3')** | **SEQ ID NO** |
|---|---|---|---|
| 1-1 | α-globin | GCTGGAGCCTCGGTGGCCATGCTTCTTGC CCCTTGGGCCTCCCCCCAGCCCCTCCTCC CCTTCCTGCACCCGTACCCCCGTGGTCTT TGAATAAAGTCTGAGTGGGCGGCA (111 bp) | 3 |
| 1-2 | β-globin | GCTCGCTTTCTTGCTGTCCAATTTCTATTA AAGGTTCCTTTGTTCCCTAAGTCCAACTA CTAAACTGGGGGATATTATGAAGGGCCT | 4 |
| | | TGAGCATCTGGATTCTGCCTAATAAAAA ACATTTATTTTCATTGCAA (134 bp) | |
| 1-3 | γ1-globin | GCTCACTGCCCATGATTCAGAGCTTTCAA GGATAGGCTTTATTCTGCAAGCAATACA AATAATAAATCTATTCTGCTGAGAGATCA CACA (90 bp) | 5 |
| 1-4 | ζ-globin | GCGCCGCCTCCGGGACCCCCAGGACAGG CTGCGGCCCCTCCCCCGTCCTGGAGGTTC CCCAGCCCCACTTACCGCGTAATGCGCCA ATAAACCAATGAACGAA (103 bp) | 6 |
| 1-5 | Tyrosine hydroxylase | GTGCACGGCGTCCCTGAGGGCCCTTCCCA ACCTCCCCTGGTCCTGCACTGTCCCGGAG CTCAGGCCCTGGTGAGGGGCTGGGTCCC GGGTGCCCCCCATGCCCTCCCTGCTGCCA GGCTCCCACTGCCCCTGCACCTGCTTCTC AGCGCAACAGCTGTGTGTGCCCGTGGTG AGGTTGTGCTGCCTGTGGTGAGGTCCTGT CCTGGCTCCCAGGGTCCTGGGGGCTGCTG CACTGCCCTCCGCCCTTCCCTGACACTGT CTGCTGCCCCAATCACCGTCACAATAAA AGAAACTGTGGTCTCTA (304 bp) | 7 |
| 1-6 | insulin | ACGCAGCCCGCAGGCAGCCCCACACCCG CCGCCTCCTGCACCGAGAGAGATGGAAT AAAGCCCTTGAACCAGC (73 bp) | 8 |

The mRNA constructs of Preparation Examples 1-1 to 1-6 designed as described above were connected a downstream side of a T7 promoter of a DNA vector (pcDNA3.1 hygro Vector), whereas an insertion was made such that a type II restriction enzyme recognition sequence was disposed immediately after the poly(A) tail of the mRNA constructs. Accordingly, the mRNA construct expressed from the DNA vector as described above was capped with CleanCap at the 5' end, and had a pure adenine sequence with a length of 117 nt at the 3' end. The DNA vector manufactured as described above was linearized by treating 5 µg of the DNA vector with the type IIS restriction enzyme BspQI (NEB Inc.) and through reactions overnight, and after identifying whether the DNA vector was completely cleaved through electrophoresis using an agarose gel, the enzyme and buffers used in the linearization process were removed by using a glassfiber membrane (Expin^{™} CleanUP SV, GeneAll Inc.), and only the linearized DNA was separated and purified. By using 500 ng of the linear DNA separated and purified as described above, in vitro transcription was carried out at 37 °C for 4 hours, and then DNase was treated and reacted at 37 °C for 30 minutes to remove the DNA used in the reaction. Then, only the mRNA generated as a result of the in vitro transcription was separated and purified by using a silica column (HiYield RNA UltraPurification Kit, RealBiotech Corporation (RBC)). The mRNA separated and purified as described above was denatured at 70 °C for 10 minutes, and then electrophoresis using an agarose gel was performed to identify that the mRNAs were uniformly generated at the expected size, and additionally, the purity and concentration of the generated mRNA were measured by using a nanodrop device (spectrophotometer, ND-2000, Thermo Scientific). Then, in a reverse-transfection method using Lipofectamine 2000^{™} Transfection Reagent (Thermo Fisher), the mRNA separated and purified as described above was transfected into 293T cells (ATCC) that had been dispensed in a 96-well plate at a density of 1×10⁴ cells/well, and the nanoluciferase activity after 6, 12, 24, 48, 72, and 96 hours of the transfection was measured by using the Nano-Glo^{®} Luciferase Assay System (Promega).

As a result, as illustrated in FIGS. 1B and 1C, it was identified that the expression of nanoluciferase was the highest and the most sustained in the mRNA construct of Preparation Example 1-4, in which the 3'-UTR of human ζ-globin was applied.

mRNA constructs of Preparation Examples 1-7 to 1-13 as illustrated in FIG. 2A were designed by applying the nucleotide sequences of ENE, which is an RNA stabilization sequence of Kaposi's sarcoma-associated herpesvirus (KSHV), and internal ribosome entry sites (IRESs) of various RNA viruses as the nucleotide sequence of the 3'-UTR.

**[Table 2]**

| **Applied preparation example #** | **Name** | **nucleotide sequence (5'->3')** | **SEQ ID NO** |
|---|---|---|---|
| 1-7 | 1X ENE | TGTTTTGGCTGGGTTCTTCCTTGTTCGCAC CGGACACCTCCAGTGACCAGACGGCAAG GTTCTTATCCCAGTGTATATTGGAAAAAC ATGTTATACTTTTGACAATTTAACGTGCC TAGAGCTCAAATTAAACTAATACCATAA CGTAATGCAACTTACAACATAAATAAAG GTCAATGTTTAATCCATA (190 bp) | 9 |
| 1-8 | 2X ENE | TGTTTTGGCTGGGTTCTTCCTTGTTCGCAC CGGACACCTCCAGTGACCAGACGGCAAG GTTCTTATCCCAGTGTATATTTGTTTTGGC TGGGTTCTTCCTTGTTCGCACCGGACACC TCCAGTGACCAGACGGCAAGGTTCTTATC CCAGTGTATATTGGAAAAACATGTTATAC TTTTGACAATTTAACGTGCCTAGAGCTCA AATTAAACTAATACCATAACGTAATGCA ACTTACAACATAAATAAAGGTCAATGTTT AATCCATA (269 bp) | 10 |
| 1-9 | 3X ENE | TGTTTTGGCTGGGTTCTTCCTTGTTCGCAC CGGACACCTCCAGTGACCAGACGGCAAG GTTCTTATCCCAGTGTATATTTGTTTTGGC TGGGTTCTTCCTTGTTCGCACCGGACACC TCCAGTGACCAGACGGCAAGGTTCTTATC CCAGTGTATATTTGTTTTGGCTGGGTTCT TCCTTGTTCGCACCGGACACCTCCAGTGA CCAGACGGCAAGGTTCTTATCCCAGTGTA TATTGGAAAAACATGTTATACTTTTGACA ATTTAACGTGCCTAGAGCTCAAATTAAAC TAATACCATAACGTAATGCAACTTACAA CATAAATAAAGGTCAATGTTTAATCCATA (348 bp) | 11 |
| 1-10 | CVB3 | TTAAAACAGCCTGTGGGTTGATCCCACCC ACAGGGCCCATTGGGCGCTAGCACTCTG GTATCACGGTACCTTTGTGCGCCTGTTTT ATACCCCCTCCCCCAACTGTAACTTAGAA GTAACACACACCGATCAACAGTCAGCGT GGCACACCAGCCACGTTTTGATCAAGCA CTTCTGTTACCCCGGACTGAGTATCAATA GACTGCTCACGCGGTTGAAGGAGAAAGC GTTCGTTATCCGGCCAACTACTTCGAAAA ACCTAGTAACACCGTGGAAGTTGCAGAG TGTTTCGCTCAGCACTACCCCAGTGTAGA TCAGGTCGATGAGTCACCGCATTCCCCAC GGGCGACCGTGGCGGTGGCTGCGTTGGC GGCCTGCCCATGGGGAAACCCATGGGAC GCTCTAATACAGACATGGTGCGAAGAGT CTATTGAGCTAGTTGGTAGTCCTCCGGCC CCTGAATGCGGCTAATCCTAACTGCGGA GCACACACCCTCAAGCCAGAGGGCAGTG TGTCGTAACGGGCAACTCTGCAGCGGAA CCGACTACTTTGGGTGTCCGTGTTTCATT TTATTCCTATACTGGCTGCTTATGGTGAC AATTGAGAGATTGTTACCATATAGCTATT GGATTGGCCATCCGGTGACTAATAGAGC TATTATATATCCCTTTGTTGGGTTTATACC ACTTAGCTTGAAAGAGGTTAAAACATTA CAATTCATTGTTAAGTTGAATACAGCAAA (742 bp) | 12 |
| 1-11 | EMCV | CCCCTCTCCCTCCCCCCCCCCTAACGTTA CTGGCCGAAGCCGCTTGGAATAAGGCCG GTGTGCGTTTGTCTATATGTTATTTTCCAC CATATTGCCGTCTTTTGGCAATGTGAGGG CCCGGAAACCTGGCCCTGTCTTCTTGACG AGCATTCCTAGGGGTCTTTCCCCTCTCGC CAAAGGAATGCAAGGTCTGTTGAATGTC GTGAAGGAAGCAGTTCCTCTGGAAGCTT CTTGAAGACAAACAACGTCTGTAGCGAC CCTTTGCAGGCAGCGGAACCCCCCACCT GGCGACAGGTGCCTCTGCGGCCAAAAGC CACGTGTATAAGATACACCTGCAAAGGC GGCACAACCCCAGTGCCACGTTGTGAGT TGGATAGTTGTGGAAAGAGTCAAATGGC TCTCCTCAAGCGTATTCAACAAGGGGCTG AAGGATGCCCAGAAGGTACCCCATTGTA TGGGATCTGATCTGGGGCCTCGGTGCAC ATGCTTTACATGTGTTTAGTCGAGGTTAA AAAACGTCTAGGCCCCCCGAACCACGGG GACGTGGTTTTCCTTTGAAAAACACGATG ATAATATGGCCACAACC (586 bp) | 13 |
| 1-12 | HCV | GGCGACACTCCACCATAGATCACTCCCCT GTGAGGAACTACTGTCTTCACGCAGAAA GCGTCTAGCCATGGCGTTAGTATGAGTGT CGTGCAGCCTCCAGGACCCCCCCTCCCGG GAGAGCCATAGTGGTCTGCGGAACCGGT GAGTACACCGGAATTGCCAGGACGACCG GGTCCTTTCTTGGATTAACCCGCTCAATG CCTGGAGATTTGGGCGTGCCCCCGCGAG ACTGCTAGCCGAGTAGTGTTGGGTCGCG AAAGGCCTTGTGGTACTGCCTGATAGGG TGCTTGCGAGTGCCCCGGGAGGTCTCGTA GACCGTGCATCATGAGCACAAATCCTAA ACCTCAAAGAAAAACC (357 bp) | 14 |
| 1-13 | CrPV | AAAGCAAAAATGTGATCTTGCTTGTAAA TACAATTTTGAGAGGTTAATAAATTACAA GTAGTGCTATTTTTGTATTTAGGTTAGCT ATTTAGCTTTACGTTCCAGGATGCCTAGT GGCAGCCCCACAATATCCAGGAAGCCCT CTCTGCGGTTTTTCAGATTAGGTAGTCGA AAAACCTAAGAAATTTA (189 bp) | 15 |

The mRNA constructs of Preparation Examples 1-7 to 1-13 designed as described above were inserted into a DNA vector in the same manner as the mRNA constructs of Preparation Examples 1-1 to 1-6, followed by in vitro transcription, and the generated mRNAs were transfected into 293T cells to measure nanoluciferase activity. As a result, as illustrated in FIGS. 2B and 2C, it was identified that the expression of nanoluciferase was the highest and the most sustained in the mRNA construct of Preparation Example 1-11, in which the IRES of EMCV was applied.

According to a recent report that mRNA stability may be further enhanced in the case of a poly(A) tail, in which guanosine (G) is mixed, compared with the case of a poly(A) tail consisting only of adenosine (A), mRNA constructs of Preparation Examples 2-1 to 2-6 as illustrated in FIG. 3A were designed by applying various nucleotide sequences disclosed in Table 3 as the nucleotide sequence of the poly(A) tail, except for the nucleotide sequence of the 3'-UTR, in the mRNA construct of Embodiment 1.

**[Table 3]**

| **Applied preparation example #** | **Name** | **nucleotide sequence (5'->3')** | **SEQ ID NO** |
|---|---|---|---|
| 2-1 | A10-G11 | A9GA10GA10GA10GA10GA10GA10GA10GA10GA10 GA10GAA | 16 |
| 2-2 | A20-G6 | A₁₃GA₂₀GA₂₀GA₂₀GA₂₀GA₂₀GAA | 17 |
| 2-3 | A30-G4 | A₂₅GA₃₀GA₃₀GA₃₀GAA | 18 |
| 2-4 | A40-G3 | A₃₆GA₄₀GA₄₀GAA | 19 |
| 2-5 | A50-G3 | A₁₆GA₅₀GA₅₀GAA | 20 |
| 2-6 | A60-G2 | A₅₇GA₆₀GAA | 21 |

The mRNA constructs of Preparation Examples 2-1 to 2-6 designed as described above were inserted into DNA vectors in the same manner as in Embodiment [2-1], followed by in vitro transcription, and the resulting mRNAs were transfected into 293T cells to measure NanoLuciferase activity. As a result, as illustrated in FIGS. 3B and 3C, among the various sequences, it was identified that the mRNA construct of Preparation Example 2-6, to which a modified poly(A) tail (A60-G2) with two guanosines (G) were inserted at intervals of 60 nt was applied, exhibited the highest and longest-lasting NanoLuciferase expression.

### [2-2] Selection of optimal combination downstream of nucleotide sequence encoding target protein

Furthermore, to select the optimal combination downstream of the nucleotide sequence encoding the target protein, in the mRNA construct specified in Embodiment 1, six mRNA constructs of Preparation Examples 3-1 to 3-6 as illustrated in A of FIG. 4 were designed by applying the modified poly(A) tail (A60-G2) selected in Embodiment 2-2 as the nucleotide sequence of the poly(A) tail, and by combining and applying the nucleotide sequence of the ζ-globin 3'-UTR (nucleotide sequence of SEQ ID NO: 6) selected in Embodiment 2-1 and the nucleotide sequence of the EMCV IRES (nucleotide sequence of SEQ ID NO: 13) as the nucleotide sequence of the 3'-UTR.

The mRNA constructs of Preparation Examples 3-1 to 3-6 designed as described above were inserted into DNA vectors in the same manner as in Embodiment [2-1], followed by in vitro transcription, and the resulting mRNAs were transfected into 293T cells to measure NanoLuciferase activity.

As a result, as illustrated in FIGS. 4B and 4C, it was identified that the expression of NanoLuciferase was the highest and the most sustained in the mRNA construct of Preparation Example 3-4, in which the 3'-UTR of ζ-globin was applied alone. Furthermore, in the case combined with the EMCV IRES, it was identified that excellent sustained expression of NanoLuciferase could be achieved in the mRNA construct of Preparation Example 3-5, in which the EMCV IRES was disposed upstream of the 3'-UTR of ζ-globin.

### [2-3] Selection of optimal 5'-UTR

Meanwhile, to derive the optimal 5'-UTR, in the mRNA construct specified in Embodiment 1, ten mRNA constructs of Preparation Examples 4-1 to 4-10 as illustrated in A of FIG. 5 were designed by fixing the 3'-UTR as the nucleotide sequence of the ζ-globin 3'-UTR (mRNA constructs of Preparation Examples 4-1 to 4-5) or the combination of the nucleotide sequence of the EMCV IRES and the ζ-globin 3'-UTR (mRNA constructs of Preparation Examples 4-6 to 4-10), and by fixing the poly(A) tail as the modified poly(A) tail A60-G2, and applying various 5'-UTR nucleotide sequences disclosed in Table 4 as the nucleotide sequence of the 5'-UTR.

**[Table 4]**

| **Applied preparation example #** | **Name** | **nucleotide sequence (5'->3')** | **SEQ ID NO** |
|---|---|---|---|
| 4-1, 4-6 | 1-23b | CAGCGGAAACGAGCGAAAAAAAAACAG CGGAAACGAGCGAAAAAAAAACAGCGG AAACGAGCGAAAAAAAAACAGCGGAAA CGAGCGAAAAAAAAACAGCGGAAACGA GCGAGAGAGAACCCCTTAAGACC (131 bp) | 22 |
| 4-2, 4-7 | 17.2 | TCCGGTCGTAAAAAAAAATCCGGTCGTA AAAAAAAATCCGGTCGTAAAAAAAAATC CGGTCGTAAAAAAAAATCCGGTCGTAGA GAGAACCCCTTAAGACC (101 bp) | 23 |
| 4-3, 4-8 | BBV | GTTTTCGAAACAAATAAAACAGAAAAGC GAACCTAAACA (39 bp) | 24 |
| 4-4, 4-9 | PPRV | AGGGGCCAAGTCCATACGACAGGCCCCA GCCCGCCAGACGGAGGGCCGCAGAAAGG AAGGAGACACGACCGCCCCAGAAGGAG GA (85 bp) | 25 |
| 4-5, 4-10 | ΔPPRV | GCCCGCCAGACGGAGGGCCGCAGAAAGG AAGGAGACACGACCGCCCCAGAAGGAG GA (57 bp) | 26 |

As a result, as illustrated in FIGS. 5B and 5C, it was identified that, in both the case, in which the nucleotide sequence of the ζ-globin 3'-UTR was used as the 3'-UTR, and the case, in which the combination of the nucleotide sequence of the EMCV IRES and the ζ-globin 3'-UTR was used as the 3'-UTR, the mRNA constructs of Preparation Examples 4-3 and 4-8, to which the nucleotide sequence of the BBV 5'-UTR was applied, and the mRNA constructs of Preparation Examples 4-4, 4-5, 4-9, and 4-10, to which the nucleotide sequence of the PPRV 5'-UTR was applied, exhibited the highest and longest-lasting NanoLuciferase expression.

### [Example 3]

### Derivation and evaluation of optimal mRNA construct

By combining the optimal elements selected in Embodiment 2, three mRNA constructs (BBV+ζG+A60G2; CJ-1, PPRV+ζG+A60G2; CJ-2, BBV+EMCV+ζG+A60G2; CJ-3) as illustrated in A of FIG. 6 were produced in the mRNA construct specified in Embodiment 1 by applying i) the nucleotide sequence of the BBV 5'-UTR (SEQ ID NO: 24) or the nucleotide sequence of the PPRV 5'-UTR (SEQ ID NO: 25) as the 5'-UTR, ii) the nucleotide sequence of the ζ-globin 3'-UTR (SEQ ID NO: 6) or the combination of the nucleotide sequence of the EMCV IRES and the nucleotide sequence of the ζ-globin 3'-UTR (SEQ ID NOs: 13+6) as the 3'-UTR, and iii) the modified poly(A) tail A60-G2 (SEQ ID NO: 21) as the poly(A) tail. Furthermore, the three mRNA constructs as described above were evaluated by comparing the mRNA constructs of Modema and Pfizer-BioNTech that have been commercially available.

First, considering the case, in which the mRNA construct is applied to vaccine development, three mRNAs generated in the same manner as in Embodiment 2-1, and the mRNAs of Modema and Pfizer-BioNTech generated in a similar manner to Embodiment 2-1 but by linearizing DNA using the type IIS restriction enzyme BsmBI (NEB) followed by in vitro transcription, were transfected into THP-1 cells (ATCC), which are representative antigen-presenting cells and monocyte-derived cells, mouse DC2.4 cells (Merck), which are dendritic cell lineages that are antigen-presenting immune cells and play important roles in muscle tissue, or cells (activated DC2.4 cells) prepared by treating DC2.4 cells with LPS, and the activity of NanoLuciferase was compared over time. As a result, as illustrated in FIGS. 6B, 6C, and 6D, it was identified that the three mRNA constructs of the present disclosure generally exhibited expression efficiency that is similar to or higher than that of the mRNA constructs of Modema and Pfizer-BioNTech, it was identified that the high efficiency of the mRNA constructs of the present disclosure was more prominently observed in DC2.4 cells, and it is identified that, in particular, the mRNA construct CJ-1 exhibited the highest efficiency.

Furthermore, considering the case, in which the mRNA construct may be applied to gene therapy for muscle diseases, liver diseases, lung diseases, eye diseases, or neurological diseases in the future, the mRNAs generated in the same manner as in Embodiment 2-1 were transfected into C2C12 cells (ATCC) as representative muscle cells, Huh-7 cells (ATCC) and HepG2 cells (ATCC) as representative liver cells, A549 cells (ATCC) as representative lung cells, ARPE-19 cells (ATCC) as representative retinal cells, SH-SY5Y cells (ATCC) and Neuro-2a cells (ATCC) as representative neuronal cells, and 293T cells, and the activity of NanoLuciferase was compared over time. As a result, as illustrated in FIGS. 7 and 8, it was identified that the mRNA constructs of the present disclosure exhibited high expression efficiency in all cells. In particular, it was identified that the mRNA constructs of Modema and Pfizer-BioNTech exhibited different expression patterns depending on the type of cells used, but the three mRNA constructs of the present disclosure consistently exhibited high expression efficiency regardless of the type of cells.

The preferred embodiments of the present disclosure have been described above by way of example, but the scope of the present disclosure is not limited to the specific embodiments described above, and those skilled in the art will be able to make appropriate modifications within the scope set forth in the claims of the present disclosure.

## Claims

1. The mRNA construct comprising:
a nucleotide sequence encoding a target protein;
a nucleotide sequence of a ζ-globin 3'-UTR disposed downstream of the nucleotide sequence encoding the target protein; and
a nucleotide sequence comprising (A₆₀G)n repeat units disposed downstream of the nucleotide sequence of the ζ-globin 3'-UTR (wherein n is an integer from 1 to 3).

2. The mRNA construct of claim 1, wherein the nucleotide sequence of the ζ-globin 3'-UTR comprises a sequence of SEQ ID NO: 6.

3. The mRNA construct of claim 1, wherein the nucleotide sequence comprising the (A₆₀G)ₙ repeat units comprises a sequence of SEQ ID NO: 21.

4. The mRNA construct of claim 1, further comprising:
a nucleotide sequence of an internal ribosome entry site (IRES) of an encephalomyocarditis virus (EMCV) between a downstream side of the nucleotide sequence encoding the target protein and an upstream side of the nucleotide sequence of the ζ-globin 3'-UTR.

5. The mRNA construct of claim 4, wherein the nucleotide sequence of the IRES of the EMCV further comprises a sequence of SEQ ID NO: 13.

6. The mRNA construct of any one of claims 1 to 5, further comprising:
a nucleotide sequence of a 5'-UTR of a black beetle virus (BBV) or a 5'-UTR of a peste des petits ruminants virus (PPRV), disposed upstream of the nucleotide sequence encoding the target protein.

7. The mRNA construct of claim 6, wherein the nucleotide sequence of the 5'-UTR of the BBV comprises a sequence of SEQ ID NO: 24, and
the nucleotide sequence of the 5'-UTR of the PPRV comprises the sequence of SEQ ID NO: 25 or a sequence of SEQ ID NO: 26.

8. The mRNA construct of claim 6, wherein the mRNA construct comprises the nucleotide sequence of the 5'-UTR of the BBV disposed upstream of the nucleotide sequence encoding the target protein.

9. The mRNA construct of claim 6, wherein the mRNA construct is for being introduced into cells by transfection.

10. A pharmaceutical composition for preventing or treating microbial infectious diseases, comprising the mRNA construct of claim 1.

11. The pharmaceutical composition of claim 10, wherein the pharmaceutical composition is a vaccine.

12. A pharmaceutical composition for preventing or treating cancer or genetic diseases, comprising the mRNA construct of claim 1.

13. The pharmaceutical composition of claim 12, wherein the pharmaceutical composition is a gene therapeutic agent.
